# EUROPEAN PATENT APPLICATION

(11) **EP 2 160 976 A1**
(43) Date of publication of application: **10.03.2010**
(21) Application number: 09169776.3
(22) Date of filing: 08.09.2009
(51) Int. Cl.: A61B 5/044

(54) **Apparatus for processing data derived from a heart pulse monitoring device**

(30) Priority: 08.09.2008 EP 08015791
(71) Applicant: Saad Specialist Hospital Co., Al-Khobar 31952 (SA)
(72) Inventor: Holzhausen, Rudolf Johannes, 31952, Al-Khobar (SA)
(74) Representative: Land, Addick Adrianus Gosling

(57) **Abstract**

The invention relates to an apparatus for processing data derived from a heart pulse monitoring device. The apparatus for processing data derived from a heart pulse monitoring device according to the invention comprises data acquisition means capable of acquiring a set of data representing a predetermined interval of said heart pulse, optionally an analog to digital conversion means for providing digital data, processing means for processing said digital data for assigning a coding to said set of data, and display means for displaying said set of data and said indicative coding.

## Description

The invention relates to an apparatus for processing data derived from a heart pulse monitoring device. Known apparatus for processing data derived from a heart pulse monitoring device such as Electrocardiographs in emergency and intensive care areas in hospitals have changed little in the past decade with respect to monitoring capability. Progress with alarm detection and interpretation such as the FA algorithm (Clifford, 2006) has greatly improved but in essence the functionality and parameters remain the same.

A drawback of the known Electrocardiographs is that possible abnormalities and / or deviations from a standard or normal expected ECG-trace produced by the electrical activity and conduction system of the heart can only be readily identified by highly specialized and experienced practitioners of Cardiology.

The invention has for its object to obviate or at least alleviate this drawback.

For this purpose the apparatus for processing data derived from a heart pulse monitoring device according to the invention comprises data acquisition means capable of acquiring a set of data representing a predetermined interval of said heart pulse, optionally an analog to digital conversion means for providing digital data, processing means for processing said digital data for assigning a coding to said set of data, and display means for displaying said set of data and said indicative coding.

This apparatus according to the invention makes it possible to clearly indicate by means of said indicative coding possible abnormalities and / or deviations from a standard or normal expected ECG trace produced by the electrical activity and conduction system of the heart.

This has the advantage for instance that non specialized medical staff are helped to indentify indications that normally would only be able to be identified by highly specialized and experienced practitioners of Cardiology.

In a preferred embodiment of the apparatus according to the invention said set of data is real-time data and said display means displays said set of data and said indicative coding real-time. By applying indicative coding on the real-time, that is live ECG trace or waveform the apparatus can assist the healthcare provider with analysis, diagnosis and observations of current medical events. For instance Cardiac Infarction (heart attack) patients are among the most vulnerable patients, as even a few minutes of delay in treatment can literally mean the difference between life and death. This apparatus brings forward the process of screening current medical events and therefore reduces diagnostic time. The real-time set of data could also be acquired from a live playback of a recorded ECG trace. The apparatus may then be unleashed as a training and / or educational tool.

In a further embodiment of the apparatus according to the invention said indicative coding is a color-coding. This makes it possible to show abnormalities and deviations as a color difference to the standard ECG-trace.

In a further embodiment of the apparatus according to the invention said processing means is designed to process said digital data by means of mathematical algorithms, preferably predictive and dynamic assessment algorithms. This approach eliminates the need for comparative analysis and/or historically based evaluation methods using for instance databases.

In a further embodiment of the apparatus according to the invention said predetermined interval of said heart pulse is 0,1 to 50 ms, preferably 0,1 to 20 ms.

The present invention will be further elucidated hereinbelow on the basis of a number of exemplary embodiments which are shown schematically in the accompanying figures. These are non-limitative exemplary embodiments. In the figures:
- Fig. 1 shows schematically the apparatus according to the invention;
- Fig. 2 shows the electrical conduction system of the heart;
- Fig. 3a shows a standard ECG trace without indicative color-coding;
- Fig. 3b shows the standard ECG trace of figure 3a with indicative color-coding of the PR-section;
- Fig. 3c shows the standard ECG trace of figure 3a with indicative color-coding of the RS-section;
- Fig. 3d shows the standard ECG trace of figure 3a with indicative color-coding of the ST-section;
- Fig. 3e shows the standard ECG trace of figure 3a with indicative color-coding of the T-wave-section;
- Fig. 4a shows a typical representation of a pathological myocardial ischemia without indicative color-coding;
- Fig. 4b shows the trace of figure 4a with color-coding indicating the deviation from normal;
- Figs. 5a-c show three subsequent moments in time of an ECG trace being color-coded real-time.

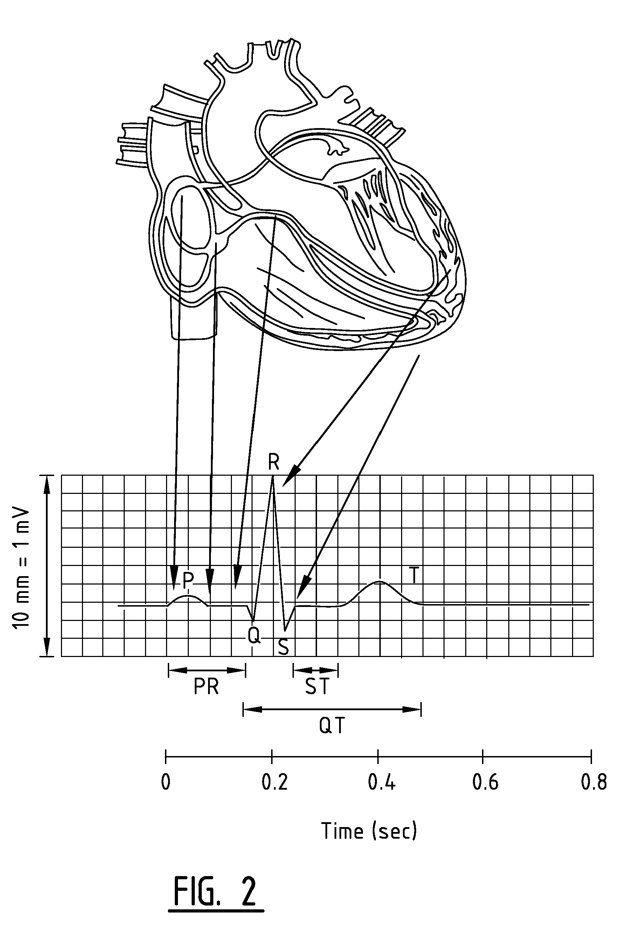

In the figures 2 to 5 color-coding is shown as thicker lines. Apart from indicative coding being color-coding, the indicative coding could also be of another form, e.g. double line, striped line etc.

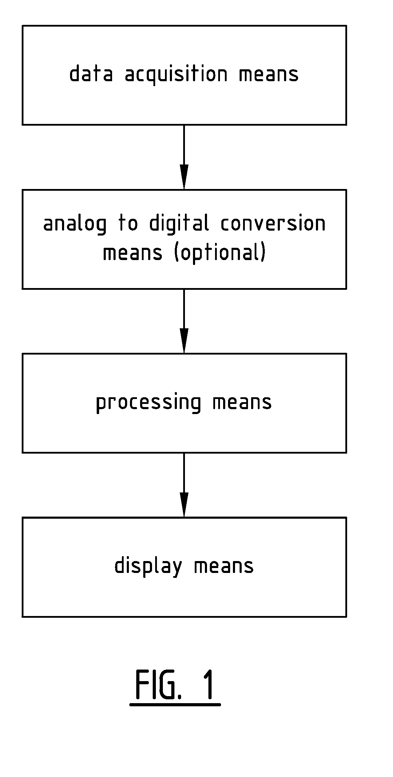

Fig. 1 shows schematically the apparatus for processing data derived from a heart pulse monitoring device according to the invention comprising data acquisition means capable of acquiring a set of data representing a predetermined interval of said heart pulse, optionally analog to digital conversion means for providing digital data, processing means for processing said digital data for assigning an indicative coding to said set of data, and display means for displaying said set of data and said indicative coding. The analog to digital conversion means is only necessary when the acquired a set of data is analog data. The heart pulse monitoring device from which the data acquisition means acquires the set of data could be a sensor placed on a patient or could be an apparatus playing back a recorded ECG trace.

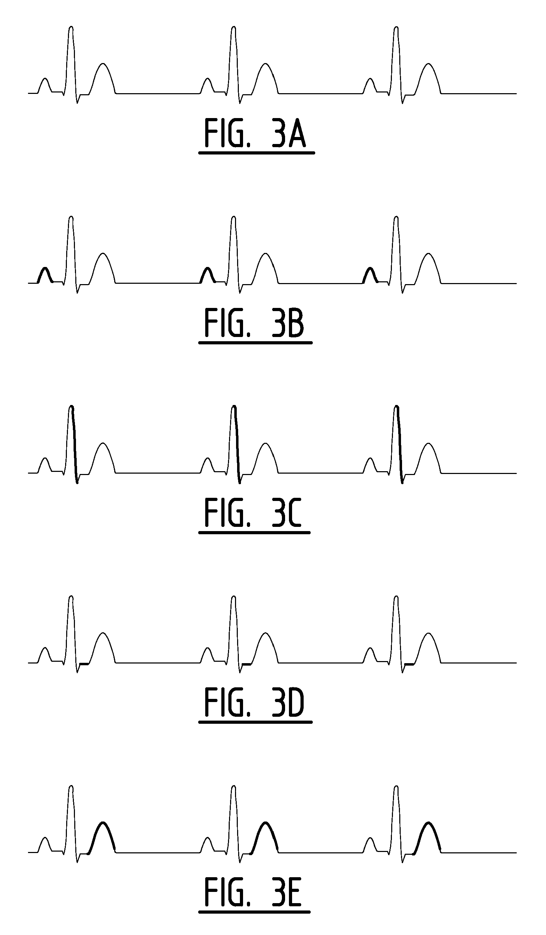

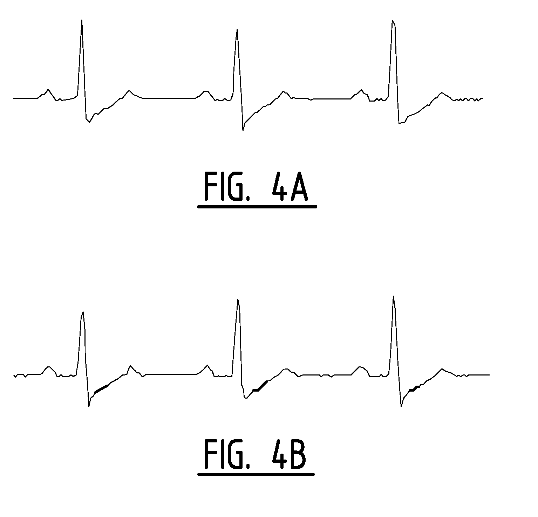

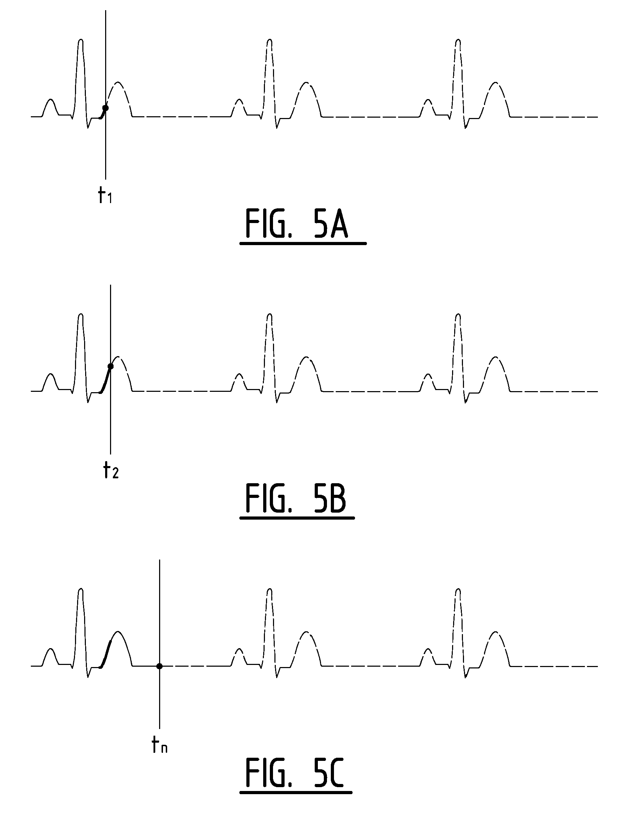

During real-time monitoring acquired sets of data are continually processed by the processing means for assigning a indicative coding to said set of data. Said processing means designed to detect and color-code arrhythmias, deviations and anomalies as can be seen in figures 4 and 5. The user has the option to enable or disable the color-coding. The indicative color-coding is not only applicable to abnormalities but may also be used for indicating sections of the clinically defined ECG trace as shown in figure 3. The user is able to select a section of the trace to be highlighted as can be seen in figures 3a-e. This enables clinicians and healthcare providers to easily identify sub-sections of the ECG trace or waveform that can simplify clinical and pathological diagnosis.

Figures 5a-c show three subsequent moments in time t₁, t₂, tₙ of an ECG trace being color-coded real-time.

## Claims

1. Apparatus for processing data derived from a heart pulse monitoring device, comprising:
data acquisition means capable of acquiring a set of data representing a predetermined interval of said heart pulse;
optionally an analog to digital conversion means for providing digital data;
processing means for processing said digital data for assigning an indicative coding to said set of data; and
display means for displaying said set of data and said indicative coding.

2. Apparatus according to claim 1, wherein said set of data is real-time data and said display means displays said set of data and said indicative coding real-time.

3. Apparatus as claimed in any of the foregoing claims, wherein said indicative coding is a color-coding

4. Apparatus as claimed in any of the foregoing claims, wherein said processing means is designed to process said digital data by means of mathematical algorithms, preferably predictive and dynamic assessment algorithms.

5. Apparatus as claimed in any of the foregoing claims wherein the length of said predetermined interval of said heart pulse is 0,1 to 50ms, preferably 0,1 to 20 ms.

6. Method for processing data derived from a heart pulse monitoring device comprising the use of an apparatus as claimed in any of the foregoing claims.
